# EUROPEAN PATENT SPECIFICATION

(11) **EP 4 551 160 B1**
(45) Date of publication and mention of the grant of the patent: **18.11.2026**
(21) Application number: 23735712.4
(22) Date of filing: 27.06.2023
(51) Int. Cl.: B22F 10/28, A61F 2/30, A61F 2/38, B33Y 80/00

(54) **PARTIALLY TRABECULAR ELEMENT FOR AUGMENT OR BONE FILLER**
TEILWEISE TRABEKULARES ELEMENT ZUR VERSTÄRKUNG ODER KNOCHENFÜLLUNG
ÉLÉMENT PARTIELLEMENT TRABÉCULAIRE À DES FINS D'AUGMENTATION OU AGENT DE REMPLISSAGE OSSEUX

(30) Priority: 07.07.2022 IT 202200014341
(43) Date of publication of application: 14.05.2025
(73) Proprietor: Limacorporate S.p.A., 33038 San Daniele del Friuli (UD) (IT)
(72) Inventor: MARTELOSSI, Marta, 34072 Gradisca d'Isonzo (GO) (IT); TONINATO, Riccardo, 33010 Colloredo di Monte Albano (UD) (IT); FIEDLER, Christoph, 18299 Diekhof (DE); PRESSACCO, Michele, 33035 Martignacco (UD) (IT)
(74) Representative: Botti & Ferrari S.p.A.
(86) International application number: PCT/EP2023/067455
(87) International publication number: WO 2024/008514

(56) References cited:
- EP-A1- 3 045 150
- WO-A1-2012/028182
- US-A1- 2007 282 455
- US-A1- 2017 333 205

## Description

### Field of application

The present invention relates to an element for augment or bone filler. In general, the present invention relates to devices that are implantable into the body, such as components for prosthesis or artificial substitutes of body parts.

### Prior art

For implantation of a prosthesis, it is possible to have to deal with an implantation site having scarcity of bone, for example because of diseases, injuries, or wear due to previous surgeries.

It is thus known to provide a porous device or element for augment, which is configured to fill a void left by a defective bone in association with a prosthesis. Similarly, bone fillers are synthetic coadjuvants that provide an osteoconductive matrix to facilitate surgeries or implantations.

However, prior art solutions are not fully effective in providing augments or bone fillers, and can therefore be improved.

Documents US 2017/0333205 A1 and EP 3 045 150 A1, which defines the features of the preamble of independent claim 1, disclose spinal implants serving as bone fillers according to the prior art.

An object of the present invention is to provide a device for an augment or bone filler that is effective.

A further object of the present invention is to allow a reduction of the total weight of an augment or bone filler, with comparable effectiveness. A further object of the present invention is to meet with the manufacturing constraints for an augment or bone filler in a qualitatively better manner.

A further object of the present invention is to provide an augment or bone filler that has a proper mechanical strength.

A further object of the present invention is to provide an augment or bone filler having structural and functional features that allow to overcome drawbacks of the prior art.

### Summary of the invention

A solution idea underlying the present invention is to provide an augment or bone filler with one or more outer layers, or "skins", made of trabecular material, such as titanium, intended to promote an osteointegration or a prosthetic cementation. Preferably, said one or more outer layers have a constant thickness. A regular reinforcing inner structure, which is associated to the one or more outer layers, allows a reduction of the total weight, thereby preserving the mechanical stability of the component.

Such reinforcing inner structure is preferably at least partially hollow and configured to allow a more effective cleaning from process powders, in a context of single-step production in particular by additive manufacturing.

Based on said solution idea, an element for augment or for bone filler is provided, comprising at least one first outer-layer portion having a trabecular lattice of metallic material, and further comprising at least one reinforcing inner layer of metallic material. The reinforcing inner layer has a plurality of channel structures arrayed with respective channel openings delimited by edges in structural contact with the at least one first outer-layer portion having the trabecular lattice.

Advantageously, the element of the present invention allows a reduction of the total weight of an augment or bone filler.

Advantageously, the element of the present invention allows to maintain the manufacturing constraints for an augment or bone filler, in particular made entirely and simultaneously by additive manufacturing, in a qualitatively better manner.

Advantageously, the element of the present invention makes it possible to remove more adequately the powder of the production process avoiding that said powder gets trapped in the porous structure of the trabecular lattice.

Advantageously, the element of the present invention allows to provide an augment or bone filler that has increased mechanical strength.

Advantageously, the element of the present invention also entails a physiological and/or psychological advantage for the final user (the patient receiving the augment or bone filler) who gets a light and non-cumbersome solution.

Preferably, the trabecular lattice of the element has a cell porosity with a first dimension, whereas the channel structures have channel equivalent diameter with a second dimension that is greater than the first dimension of the trabecular cell porosity. Moreover, preferably, the channel structures comprise respective through channel cavities. In this way, advantageously, a powder or other production residues, in particular in the case of additive manufacturing, can pass through the trabecular lattice and be easily evacuated through the channel structures, in particular aided by gravitational effects. In this way, advantageously, a cleaning effect contributes to providing a high-quality element.

Preferably, the channel structures are adjoining and substantially aligned, in particular defining a honeycomb structure, so as to contribute to the mechanical strength of the element.

Preferably, the channel structures are partially diverging from each other, to follow a slanted contour surface of the element. Advantageously, in this way an effect of better cleaning as described above, and possibly a better load transmission through the element, is obtained. In particular, advantageously, the channel structures are arrayed along a prevalent stress direction of the element.

Preferably, the element further comprises at least one second outer-layer portion having the trabecular lattice of metallic material and associated to the plurality of channel structures. Advantageously, in this way prosthetic cementation or osteointegration is improved.

Preferably, the element further comprises at least one third outer-layer portion with bulk thin shell of metallic material configured to at least partially surround the element laterally. Said third outer-layer portion is particularly suitable for contact with soft tissues.

The element is made entirely and simultaneously by additive manufacturing, so as to obtain structural continuity between the at least one first outer-layer portion and the at least one reinforcing inner layer, and a resulting improvement of mechanical performances of the element. The element is entirely made of metallic material by means of additive manufacturing. Preferably, the metallic material includes titanium for improving biocompatibility.

Further features and advantages of the invention will become apparent from the following detailed description of embodiments, which is provided by way of non-limiting illustration, and from the claims defining the invention, which are integral part of the present description.

### Brief description of the drawings

- Figure 1 shows a three-dimensional view of a first embodiment of the present invention.
- Figure 2 shows a further three-dimensional view of the first embodiment of the present invention.
- Figure 3 shows a perspective top view of the first embodiment of the present invention.
- Figure 4 shows a perspective bottom view of the first embodiment of the present invention.
- Figure 5 shows a top view of the first embodiment of the present invention.
- Figure 6 shows a perspective sectional view of the first embodiment of the present invention.
- Figure 7 shows a first section of Figure 5.
- Figure 8 shows a second section of Figure 5.
- Figure 9 shows a perspective top view of a second embodiment of the present invention.
- Figure 10 shows a further perspective top view of the second embodiment of the present invention.
- Figure 11 shows a perspective sectional view of the second embodiment of the present invention.
- Figure 12 shows a sectional view of the second embodiment of the present invention.
- Figure 13 shows a top view of a third embodiment of the present invention.
- Figure 14 shows a sectional view of Figure 13.
- Figure 15 exemplifies a first application of the present invention.
- Figure 16 exemplifies a second application of the present invention.

In different figures, analogous elements will be indicated by analogous reference numerals. Frequently, if one figure contains more analogous elements, only one or some of them will be indicated by a respective reference numeral for the purpose of improved legibility, construing that also the others are included in the discussion.

### Detailed description

The elements of the present invention are meant for applications of augment or of bone filler. In this description, the focus will be on elements for augment, and it will be intended that analogous solutions are applicable to elements for bone filler.

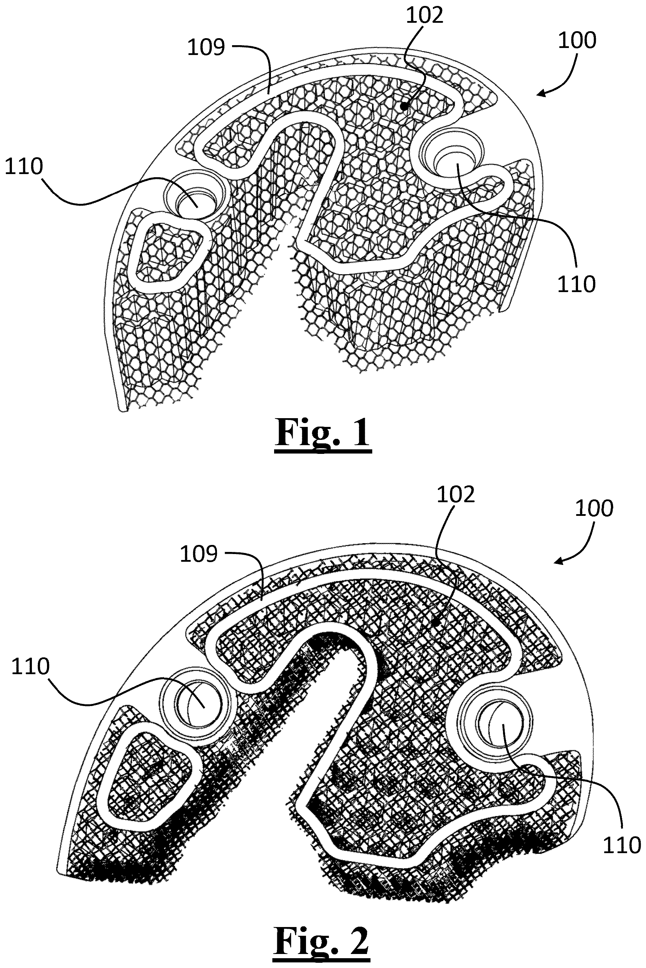

Figure 1 shows a three-dimensional view of an embodiment of element 100, which is in particular an element for augment, whereas Figure 2 shows a further three-dimensional view of the element 100, with a slightly different graphic representation of the trabecular portion.

The element 100 comprises an outer-layer portion 102 having a trabecular lattice, intended for osteointegration or prosthetic cementation. The trabecular lattice is made of metallic material. In general, the portions having trabecular lattice are particularly intended to be in contact with a bone surface.

The element 100 further comprises at least one reinforcing inner layer which has a plurality of channel structures that can be barely seen in the above-mentioned figures and that will be further described in more detail. The reinforcing inner layer is made of metallic material.

An element according to the present invention is obtainable in particular by means of additive manufacturing techniques. Preferably, SLM (Selective Laser Melting) processes are used, which represent the enabling reference technology. Alternatively, EBM (Electron Beam Melting) processes are used.

Specific embodiments of elements according to the present invention provide for a manufacturing process being a metal additive process of powder bed fusion, in particular E-PBF (Electron-beam Powder Bed Fusion) and L-PBF (Laser Powder Bed Fusion).

The element is in particular of metallic material comprising titanium.

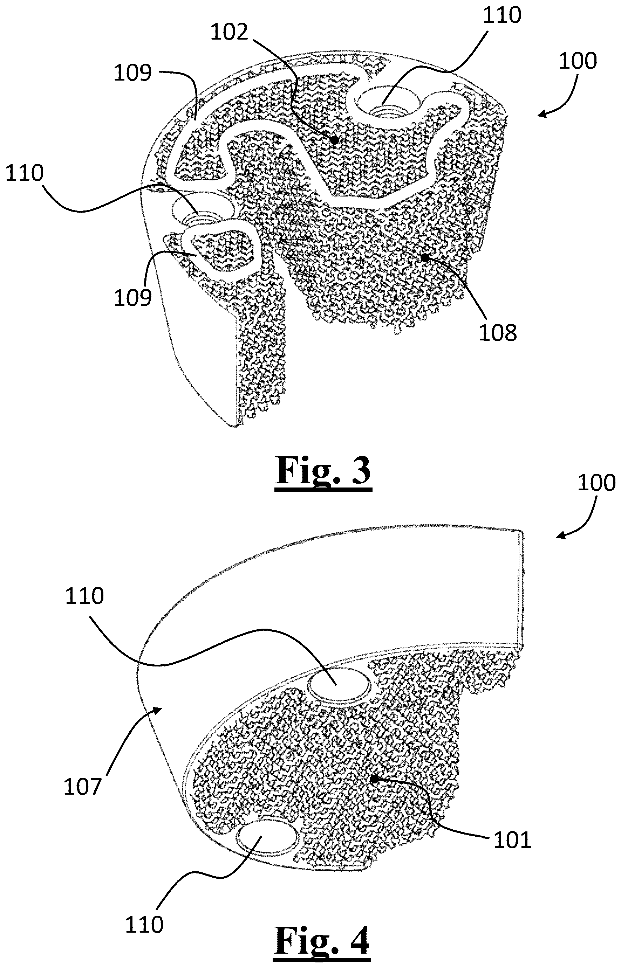

Figure 3 shows a perspective top view of the element 100, in which the outer-layer portion 102 is visible, that will be further described in the following. In this figure and in the next ones, the graphic representation for the trabecular portion is slightly different than the previous figures.

Figure 4 shows a perspective bottom view of the element 100. The element 100 comprises at least one first outer-layer portion 101 having the trabecular lattice of metallic material. In general, the at least one first outer-layer portion 100 having the trabecular lattice occupies at least 45% of a surface of a respective side of the element. In this example, the trabecular portion substantially occupies the whole surface of the corresponding side.

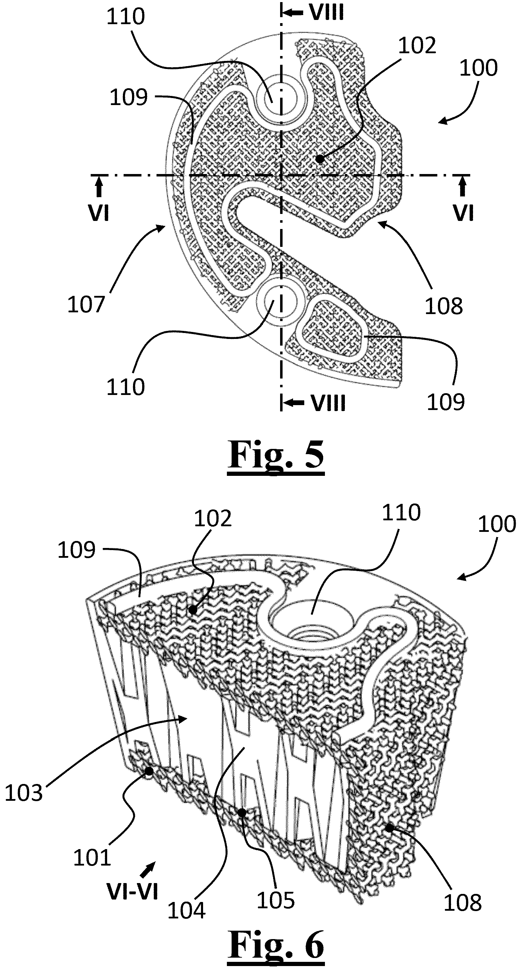

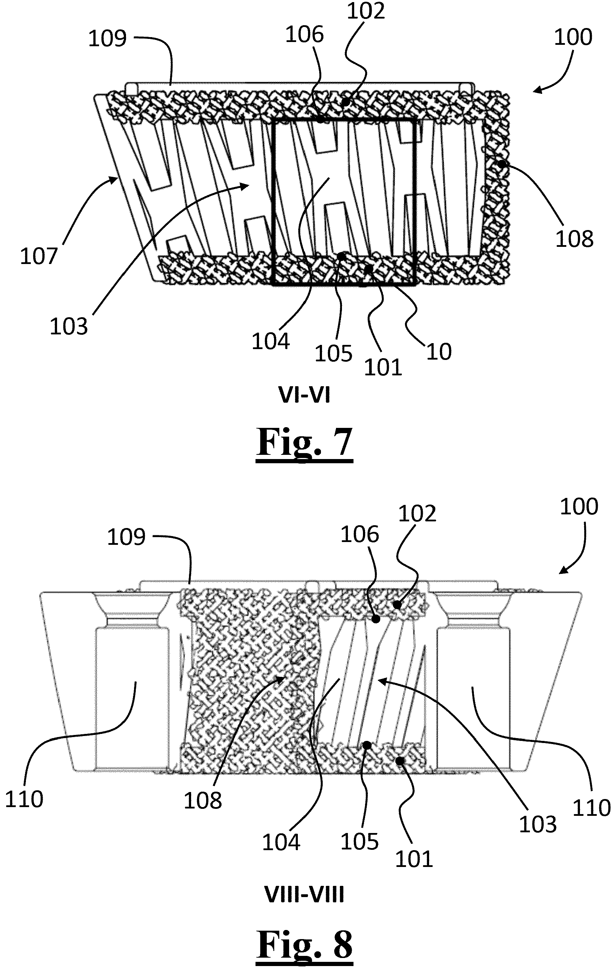

Figure 5 shows a top view of the element 100, in which two sections VI and VIII are observed. Figure 6 shows a perspective view along section VI of the element 100 and Figure 7 shows a planar view of the element 100 along section VI.

In these views it is possible to observe, especially inside the illustrative box 10 of Figure 7, that the element 100 comprises at least one first outer-layer portion 101 having a trabecular lattice of metallic material.

The element 100 further comprises at least one reinforcing inner layer 103 of metallic material. The reinforcing inner layer 103 has a plurality of channel structures 104. The channel structures 104 are arrayed with respective channel openings 105 delimited by edges in structural contact with the at least one first outer-layer portion 101.

For an element 100 made entirely and simultaneously of metallic material by additive manufacturing, not only there is structural contact between the channel structures 104 and the first outer-layer portion 101, but there is a structural continuity at least between the at least one first outer-layer portion 101 and the at least one reinforcing inner layer 103.

Preferably, the trabecular lattice of the first outer-layer portion 101 and of the further areas comprising the trabecular lattice has a cell porosity with a first dimension.

The channel structures 104 have a channel equivalent diameter with a second dimension, that is greater than the first dimension of the cell porosity of the trabecular lattice. Simply put, the channel structures 104 are, in the diametral or transversal direction, larger than the porosity or than the dimension characteristic of the cell of the trabecular lattice. In this way, it becomes easier to evacuate possible powders that are weakly adherent or also processing residues, to the advantage of a better de-powdering in a context of additive manufacturing.

Preferably, the channel structures 104 are adjoining and substantially aligned. In this preferred example, the channel structures define a honeycomb structure with hexagonal cells, being it understood that different contours of the cells beside hexagonal (for example: circular, oval, polygonal) are possible. For this reason, "channel equivalent diameter" means a diameter associated with a circular contour that inscribes or circumscribes the contour of the channel structure 104, so as to approximate it.

In particularly advantageous embodiments, as it can be observed in this example, the channel structures 104 are partially diverging from each other, to follow a slanted contour surface of the element 100. In particular, the slanted contour surface of the element 100 is determined based on evaluations of the anatomy with which the element 101 is associated. "Partially diverging" means that the respective axes are not parallel to each other and therefore diverge, and possibly also that the equivalent diameter of each channel structure 104 is not constant but diverging.

In general, the outer shape of the element 101 may be anatomically designed with at least one side having inclinations between 0° and 40°. This particular design results in a stretched reinforcing structure that ensures open channels and that at the same time can optimize load distribution on the trabecular outer layers, on suitable load areas determined based on considerations on the anatomy or on prosthesis geometry. In particular, preferably, the channel structures 104 are arrayed along a prevalent stress direction of the element 101.

The reinforcing inner layer 103 has preferably regular structures that minimize the amount of material required for structural stability, so as to obtain a minimum weight and a minimum cost of material. The geometry of the channel structures 104 can vary widely, but a common feature of preferred channel structures is an array of hollow cells formed between thin walls. The hollow cells have preferably a columnar and hexagonal shape. A reinforcing inner layer 103, such as a honeycomb structure, provides a structured material with minimum density and relatively high properties of out-of plane compression and properties of out-of plane cutting. The result is a design that allows primary stability and secondary fixation, as well as suitable mechanical properties due to the inner honeycomb, also resulting extremely light.

In the herein-represented preferred embodiment, the channel structures 104 comprise respective through channel cavities.

In possible variants that are not represented, the element might comprise a further reinforcing inner layer having a bulk reinforcement, transversal to the channel structures, so as to define non-through channel cavities. These possible variants may provide a better strength to the element, even though the cleaning from powders or production residues is potentially more complex and may implicate the use of physical and/or chemical post-processing.

In this embodiment of the element 100, there is at least one second outer-layer portion 102 having the trabecular lattice of metallic material.

The at least one second outer-layer portion 102 is positioned opposite the at least one first outer-layer portion 101 which has already been described, with respect to the reinforcing inner layer 103, thereby making, so to speak, a sandwich structure.

There are respective second channel openings 106 of the plurality of channel openings 104 that are further delimited by second edges in structural contact with the at least one second outer-layer portion 102.

Preferably, the element 100 further comprises at least one third outer-layer portion 107 with bulk thin shell of metallic material, configured to at least partially surround the element 100 laterally to the reinforcing inner layer 103.

The element 100 further comprises at least one fourth outer-layer portion 108 of trabecular metallic material configured to at least partially surround the element 100 laterally to the reinforcing inner layer 103.

Moreover, preferably, the element 100 further comprises a bulk protruding profile 109 on a surface opposite the at least one first outer-layer portion 101. In particular, the bulk protruding profile 109 is configured for a positive-locking fit with a further prosthesis component, in applications for augments. For example, for the fit with a negative shape of a component, for example a tibial plate in the case of a knee, a bulk protruding profile 109 might be applied, thereby creating the required positive shape.

Instead, differently, in an application of element for bone filler the presence of bulk protruding profiles is not provided.

Figure 8 shows a further planar view of the element 100 along section VIII.

As it can be observed, the element 100 comprises at least one hole 110, configured for the insertion of a connecting screw for the connection to a further prosthesis component in applications for augments, or for the connection to the bone in the case of a bone filler.

By way of a non-limiting example, the dimensions of the element 100 can be:
- first outer-layer portion 101: thickness 1.2 - 2.0 mm
- second outer-layer portion 103: thickness 1.2 - 2.0 mm
- reinforcing inner layer 103: height 6 - 16 mm
- channel structures 104: wall thickness about 1.0 mm
- channel openings 105: equivalent diameter 3.5 - 4.0 mm
- channel openings 106: equivalent diameter 3.5 - 4.0 mm
- third outer-layer portion 107: thickness 1.0 - 2.0 mm

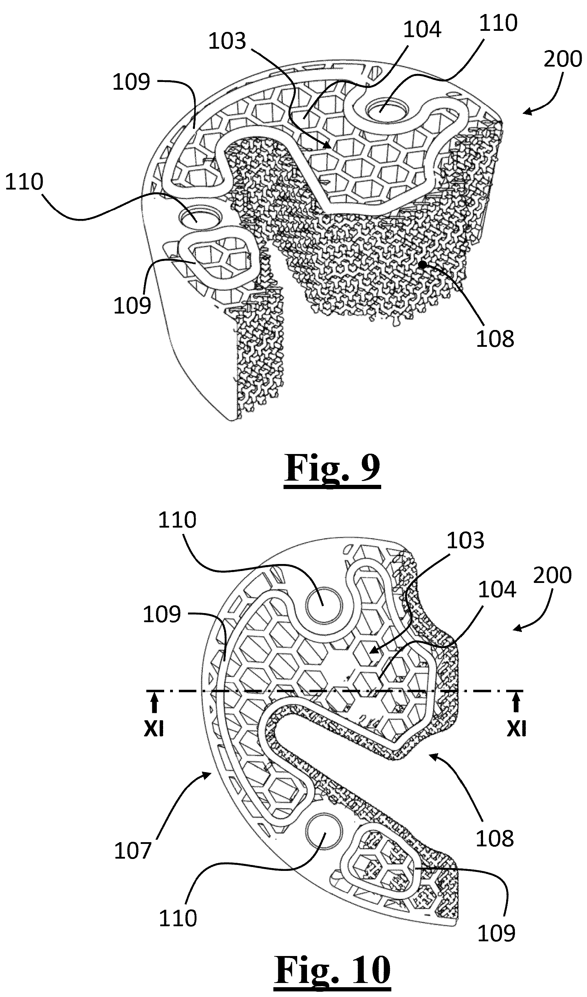

Figure 9 shows a perspective top view of a second embodiment of element 200.

Similarly to what has been described with reference to the embodiment of element 100, the element 200 for augment comprises at least one first outer-layer portion 101 having a trabecular lattice, and also at least one reinforcing inner layer 103 having a plurality of channel structures 104 arrayed with respective channel openings 105 delimited by edges in structural contact with the at least one first outer-layer portion 101.

Therefore, the element 200 corresponds to a great extent to the element 100 which has already been described. It is noteworthy that in the element 200 there is not the second outer-layer portion that was previously described, and on top of the element the channel structures 104 are directly visible.

In the embodiment of the element 200, the channel structures are directly configured to fit with a prosthesis surface, preferably by means of the bulk protruding profile 109.

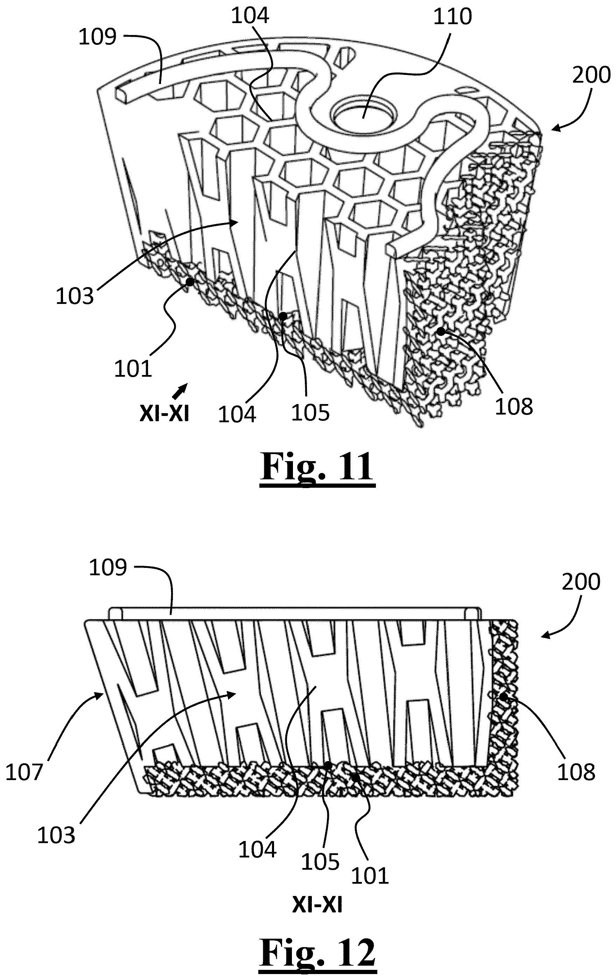

Figure 10 shows a further perspective top view of the element 200 in which section XI is observed. Figure 11 shows a perspective view of the element 200 along section XI, and Figure 12 shows a planar view of the element 200 along section XI.

In these views, it is possible to see that in the element 200, made of metallic material entirely and simultaneously by additive manufacturing, there is structural continuity at least between the at least one first outer-layer portion 101 and the at least one reinforcing inner layer 103.

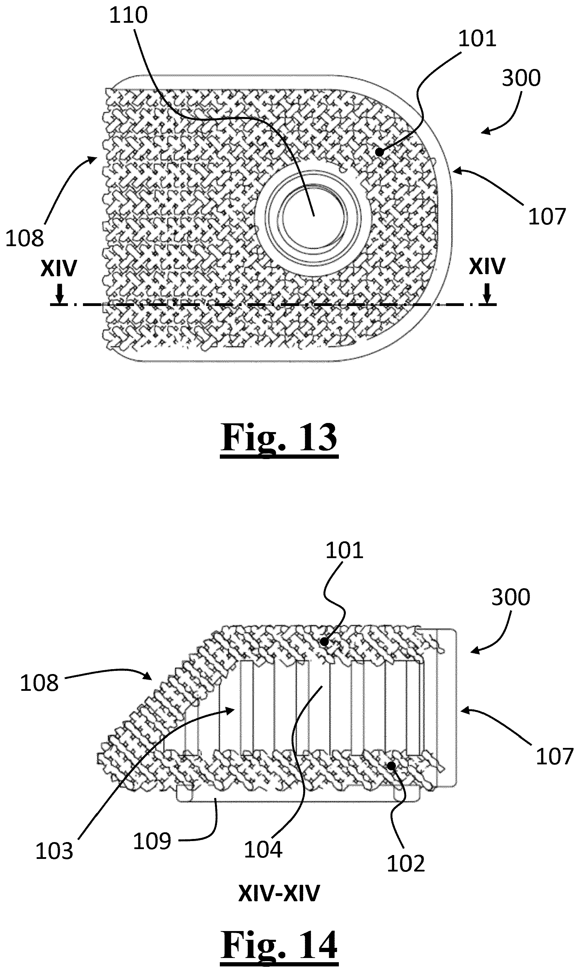

Figure 13 shows a top view of a third embodiment of element 300 in which a section XIV is observed. Figure 14 shows a planar view of the element 300 along section XIV.

Similarly to what has been described with reference to the embodiment of element 100, the element 300 for augment comprises at least one first outer-layer portion 101 having a trabecular lattice, and also at least one reinforcing inner layer 103 having a plurality of channel structures 104 arrayed with respective channel openings 105 delimited by edges in structural contact with the at least one first outer-layer portion 101.

Therefore, the element 300 corresponds to a great extent to the element 100 which has already been described. It is noteworthy that in the element 300 there are not channel structures 104 that partially diverge from each other; instead, they are parallel to each other. In particular, the respective axes of the channel structures 104 are parallel to each other and possibly also the equivalent diameter of each channel structure 104 is constant along the axial extension.

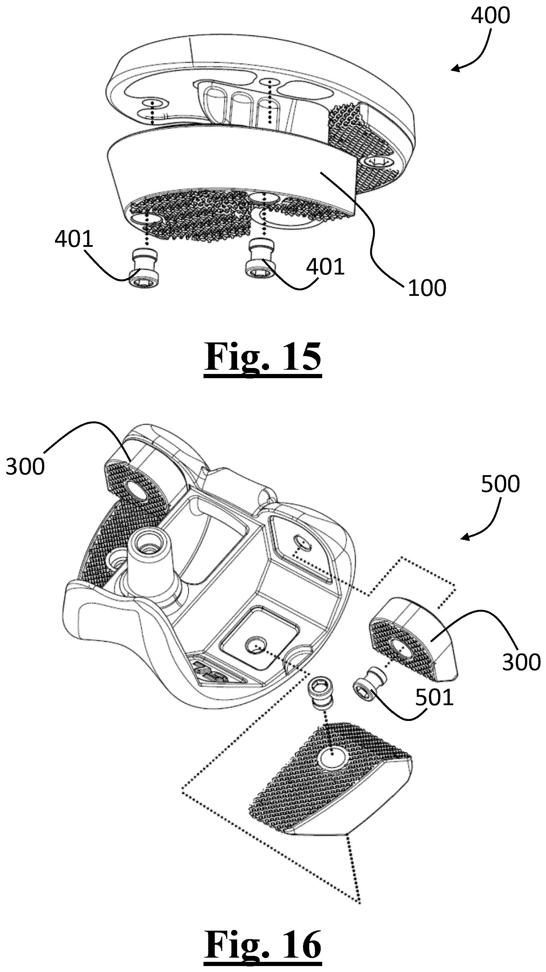

Figure 15 exemplifies a first application of an element 100, whereas Figure 16 exemplifies a second application of an element 300, for augments of prosthesis.

The tibial augment 100 and the femoral augment 300 are assembled on tibial components 400 and femoral components 500, respectively, through specific connecting screws 401 and 501.

The elements of femoral augment 300 and tibial augment 100 are used to fill bone defects of moderate dimensions with biomechanically stable components to support the weight and assist the functional movement, thereby helping to restore an anatomical articular line.

The augments are typically modular and are considered an alternative solution to bone cement, to autograft, and to structural allograft in dealing with bone loss.

The present invention combines design and process in an element of prosthesis augment or bone filler.

Furthermore, the solution allows a reduction of the weight of the total prosthesis of about 50% with respect to the same solution obtained from the solid, thereby allowing at the same time a better quality of the production process.

It is evident that further implementations and modifications of the present invention will be possible for the person skilled in the art to meet particular needs, as long as such implementations and modifications fall within the scope of the appended claims.

For example, the geometries of the elements are purely illustrative and may be adapted to the type of prosthesis or implant.

Therefore, the above-described embodiments are to be understood as provided by way of non-limiting illustration.

## Claims

1. Element (100, 200, 300) for augment or bone filler, comprising:
- at least one first outer-layer portion (101), and
- at least one reinforcing inner layer (103) of said metallic material, said at least one reinforcing inner layer (103) having a plurality of channel structures (104) arrayed with respective channel openings (105) delimited by edges in structural contact with said at least one first outer-layer portion (101),
wherein said metallic material is made entirely and simultaneously by additive manufacturing and has structural continuity at least between said at least one first outer-layer portion (101) and said at least one reinforcing inner layer (103);
**characterized in that** said at least one first outer-layer portion has a trabecular lattice of metallic material; and
**in that** it further comprises at least one fourth outer-layer portion (108) of trabecular metallic material configured to at least partially surround said element (100, 200, 300) laterally to said reinforcing inner layer (103).

2. Element according to claim 1, wherein said trabecular lattice has a cell porosity with a first dimension, and wherein said channel structures (104) have channel equivalent diameter with a second dimension greater than said first dimension.

3. Element according to claim 1 or 2, wherein said channel structures (104) are adjoining and substantially aligned, in particular defining a honeycomb structure.

4. Element according to claim 3, wherein said channel structures (104) are partially diverging from each other, to follow a slanted contour surface of said element (100, 200).

5. Element according to any one of claims 1 to 4, wherein said channel structures (104) are arrayed along a direction of said element (100, 200, 300), and wherein said element is configured to be subject to a prevalent stress in said direction.

6. Element according to any one of claims 1 to 5, wherein said channel structures (104) comprise respective through channel cavities.

7. Element according to any one of claims 1 to 5, wherein said at least one reinforcing inner layer (103) further comprises a bulk reinforcement transversal to said channel structures (104), thus defining non-through channel cavities.

8. Element according to any one of claims 1 to 7, further comprising at least one second outer-layer portion (102) having said trabecular lattice of metallic material and positioned opposite said at least one first outer-layer portion (101) with respect to said reinforcing inner layer (103), wherein respective second channel openings (106) of said plurality of channel structures (104) are further delimited by second edges in structural contact with said at least one second outer-layer portion (102).

9. Element according to any one of claims 1 to 8, further comprising at least one third outer-layer portion (107) with bulk thin shell of metallic material configured to at least partially surround said element (100, 200, 300) laterally to said reinforcing inner layer (103).

10. Element according to any one of claims 1 a 9, further comprising a bulk protruding profile (109) of metallic material on a surface opposite said at least one first outer-layer portion (101), said bulk protruding profile (109) being configured for a positive-locking fit with a further prosthesis component (400. 500).

11. Element according to any one of claims 1 to 10, further comprising at least one hole (110) configured for inserting a connecting screw (401; 501) to connect to a further prosthesis component (400; 500) or directly to a bone.

12. Element according to any one of claims 1 to 11, wherein said at least one first outer-layer portion (101) having said trabecular lattice of metallic material occupies at least 45% of a surface of a respective side of said element (100, 200, 300).

13. Element according to any one of claims 1 to 12, wherein said metallic material comprises titanium.

## Patentansprüche

1. Element (100, 200, 300) für Augmentation oder Knochenfüllung, umfassend:
- mindestens einen ersten Außenschichtabschnitt (101), und
- mindestens eine verstärkende Innenschicht (103) aus dem metallischen Material, wobei die mindestens eine verstärkende Innenschicht (103) eine Mehrzahl von Kanalstrukturen (104) aufweist, die mit jeweiligen Kanalöffnungen (105) aufgereiht sind, die durch Kanten begrenzt werden, die in strukturellem Kontakt mit dem mindestens einen ersten Außenschichtabschnitt (101) stehen,
wobei das metallische Material vollständig und gleichzeitig durch additive Fertigung hergestellt wird und strukturelle Kontinuität zumindest zwischen dem mindestens einen ersten Außenschichtabschnitt (101) und der mindestens einen verstärkenden Innenschicht (103) aufweist;
**dadurch gekennzeichnet, dass** der mindestens eine erste Außenschichtabschnitt ein Trabekelgitterwerk aus metallischem Material aufweist; und
**dass** es ferner mindestens einen vierten Außenschichtabschnitt (108) aus metallischem Trabekelmaterial umfasst, der dazu ausgelegt ist, das Element (100, 200, 300) seitlich zur verstärkenden Innenschicht (103) zumindest teilweise zu umgeben.

2. Element nach Anspruch 1, wobei das Trabekelgitterwerk eine Zellenporosität mit einer ersten Abmessung aufweist und wobei die Kanalstrukturen (104) einen äquivalenten Kanaldurchmesser mit einer zweiten Abmessung aufweisen, die größer als die erste Abmessung ist.

3. Element nach Anspruch 1 oder 2, wobei die Kanalstrukturen (104) angrenzend und im Wesentlichen fluchtend sind, insbesondere eine Wabenstruktur definieren.

4. Element nach Anspruch 3, wobei die Kanalstrukturen (104) teilweise voneinander divergieren, um einer geneigten Konturoberfläche des Elements (100, 200) zu folgen.

5. Element nach einem der Ansprüche 1 bis 4, wobei die Kanalstrukturen (104) entlang einer Richtung des Elements (100, 200, 300) aufgereiht sind und wobei das Element dazu ausgelegt ist, einer vorherrschenden Belastung in dieser Richtung ausgesetzt zu sein.

6. Element nach einem der Ansprüche 1 bis 5, wobei die Kanalstrukturen (104) jeweilige durchgehende Kanalhohlräume umfassen.

7. Element nach einem der Ansprüche 1 bis 5, wobei die mindestens eine verstärkende Innenschicht (103) ferner eine massive Verstärkung quer zu den Kanalstrukturen (104) umfasst und damit nicht durchgehende Kanalhohlräume definiert.

8. Element nach einem der Ansprüche 1 bis 7, ferner umfassend mindestens einen zweiten Außenschichtabschnitt (102), der das Trabekelgitterwerk aus metallischem Material aufweist und dem mindestens einen ersten Außenschichtabschnitt (101) in Bezug auf die verstärkende Innenschicht (103) gegenüberliegend angeordnet ist, wobei jeweilige zweite Kanalöffnungen (106) der Mehrzahl von Kanalstrukturen (104) ferner durch zweite Kanten begrenzt werden, die in strukturellem Kontakt mit dem mindestens einen zweiten Außenschichtabschnitt (102) stehen.

9. Element nach einem der Ansprüche 1 bis 8, ferner umfassend mindestens einen dritten Außenschichtabschnitt (107) mit einer massiven dünnen Schale aus metallischem Material, der dazu ausgelegt ist, das Element (100, 200, 300) seitlich zur verstärkenden Innenschicht (103) zumindest teilweise zu umgeben.

10. Element nach einem der Ansprüche 1 bis 9, ferner umfassend ein massives vorstehendes Profil (109) aus metallischem Material auf einer dem mindestens einen ersten Außenschichtabschnitt (101) gegenüberliegenden Oberfläche, wobei das massive vorstehende Profil (109) für einen Formschluss mit einer weiteren Prothesenkomponente (400, 500) ausgelegt ist.

11. Element nach einem der Ansprüche 1 bis 10, ferner umfassend mindestens ein Loch (110), das zum Einführen einer Verbindungsschraube (401; 501) ausgelegt ist, um eine Verbindung mit einer weiteren Prothesenkomponente (400; 500) oder direkt mit einem Knochen herzustellen.

12. Element nach einem der Ansprüche 1 bis 11, wobei der mindestens eine erste Außenschichtabschnitt (101), der das Trabekelgitterwerk aus metallischem Material aufweist, mindestens 45 % einer Oberfläche einer jeweiligen Seite des Elements (100, 200, 300) einnimmt.

13. Element nach einem der Ansprüche 1 bis 12, wobei das metallische Material Titan umfasst.

## Revendications

1. Élément (100, 200, 300) à des fins d'augmentation ou agent de remplissage osseux, comprenant :
- au moins une première portion de couche externe (101), et
- au moins une couche interne de renforcement (103) dudit matériau métallique, ladite au moins une couche interne de renforcement (103) ayant une pluralité de structures de canal (104) disposées en réseau avec des ouvertures de canal respectives (105) délimitées par des bords en contact structurel avec ladite au moins une première portion de couche externe (101),
dans lequel ledit matériau métallique est fabriqué entièrement et simultanément par fabrication additive et a une continuité structurelle au moins entre ladite au moins une première portion de couche externe (101) et ladite au moins une couche interne de renforcement (103) ;
**caractérisé en ce que** ladite au moins une première portion de couche externe a un treillis trabéculaire de matériau métallique ; et
**en ce qu'**il comprend en outre au moins une quatrième portion de couche externe (108) de matériau métallique trabéculaire configurée pour entourer au moins partiellement ledit élément (100, 200, 300) latéralement par rapport à ladite couche interne de renforcement (103).

2. Élément selon la revendication 1, dans lequel ledit treillis trabéculaire a une porosité cellulaire avec une première dimension, et dans lequel lesdites structures de canal (104) ont un diamètre équivalent de canal avec une seconde dimension supérieure à ladite première dimension.

3. Élément selon la revendication 1 ou 2, dans lequel lesdites structures de canal (104) sont attenantes et sensiblement alignées, en particulier définissant une structure en nid d'abeilles.

4. Élément selon la revendication 3, dans lequel lesdites structures de canal (104) divergent partiellement les unes des autres, pour le suivi d'une surface de contour inclinée dudit élément (100, 200).

5. Élément selon l'une quelconque des revendications 1 à 4, dans lequel lesdites structures de canal (104) sont disposées en réseau le long d'une direction dudit élément (100, 200, 300), et dans lequel ledit élément est configuré pour être soumis à une contrainte prédominante dans ladite direction.

6. Élément selon l'une quelconque des revendications 1 à 5, dans lequel lesdites structures de canal (104) comprennent des cavités de canal traversantes respectives.

7. Élément selon l'une quelconque des revendications 1 à 5, dans lequel ladite au moins une couche interne de renforcement (103) comprend en outre un renforcement massif transversal auxdites structures de canal (104), définissant ainsi des cavités de canal non traversantes.

8. Élément selon l'une quelconque des revendications 1 à 7, comprenant en outre au moins une deuxième portion de couche externe (102) ayant ledit treillis trabéculaire de matériau métallique et positionnée à l'opposé de ladite au moins une première portion de couche externe (101) par rapport à ladite couche interne de renforcement (103), dans lequel des secondes ouvertures de canal respectives (106) de ladite pluralité de structures de canal (104) sont en outre délimitées par des seconds bords en contact structurel avec ladite au moins une deuxième portion de couche externe (102).

9. Élément selon l'une quelconque des revendications 1 à 8, comprenant en outre au moins une troisième portion de couche externe (107) avec une coque mince massive de matériau métallique configurée pour entourer au moins partiellement ledit élément (100, 200, 300) latéralement par rapport à ladite couche interne de renforcement (103).

10. Élément selon l'une quelconque des revendications 1 à 9, comprenant en outre un profil saillant massif (109) de matériau métallique sur une surface opposée à ladite au moins une première portion de couche externe (101), ledit profil saillant massif (109) étant configuré pour un ajustement à verrouillage positif avec un composant de prothèse supplémentaire (400. 500).

11. Élément selon l'une quelconque des revendications 1 à 10, comprenant en outre au moins un trou (110) configuré pour l'insertion d'une vis de raccordement (401 ; 501) pour le raccordement à un composant de prothèse supplémentaire (400 ; 500) ou directement à un os.

12. Élément selon l'une quelconque des revendications 1 à 11, dans lequel ladite au moins une première portion de couche externe (101) ayant ledit treillis trabéculaire de matériau métallique occupe au moins 45 % d'une surface d'un côté respectif dudit élément (100, 200, 300).

13. Élément selon l'une quelconque des revendications 1 à 12, dans lequel ledit matériau métallique comprend du titane.
